# EUROPEAN PATENT APPLICATION

(11) **EP 4 108 159 A1**
(43) Date of publication of application: **28.12.2022**
(21) Application number: 21756551.4
(22) Date of filing: 18.02.2021
(51) Int. Cl.: A61B 3/06, G01N 21/27

(54) **METHOD FOR INSPECTING VISUAL CHARACTERISTICS, METHOD FOR DETERMINING CHARACTERISTICS OF OPTICAL FILTER, OPTICAL FILTER, OPTICAL ELEMENT SET FOR VISUAL CHARACTERISTIC INSPECTION, AND VISUAL CHARACTERISTIC INSPECTION IMAGE**

(30) Priority: 21.02.2020 JP 2020028739
(71) Applicant: Iris Communication Kabushiki Kaisha, Tokyo 1010031 (JP); Nico Corporation, Tokyo 162-0843 (JP)
(72) Inventor: YANO, Masafumi, Sendai-shi, Miyagi 981-8004 (JP)
(74) Representative: Ablett, Graham Keith
(86) International application number: PCT/JP2021/006094
(87) International publication number: WO 2021/166996

(57) **Abstract**

A method for testing visual characteristics includes a providing step of providing a subject with a test image and a changing step of changing an optical element to be placed between the subject and the test image. The test image includes a first image area containing a color to which rod cells of a human are sensitive, and the first image area is placed in such a manner that light coming from the first image area forms an image on a region outside a central fovea of a retina of the subject when the subject is looking at a center of the test image. The optical element set includes a plurality of first optical elements configured to transmit light of a first wavelength band to which the rod cells are sensitive within a visible light band. An optical element that the subject does not feel dazzled by the test image is specified.

## Description

### TECHNICAL FIELD

The present invention relates to a method for testing visual characteristics, a method for determining characteristics of optical filter, an optical filter, an optical element set for testing visual characteristics and a test image for testing visual characteristics.

### BACKGROUND

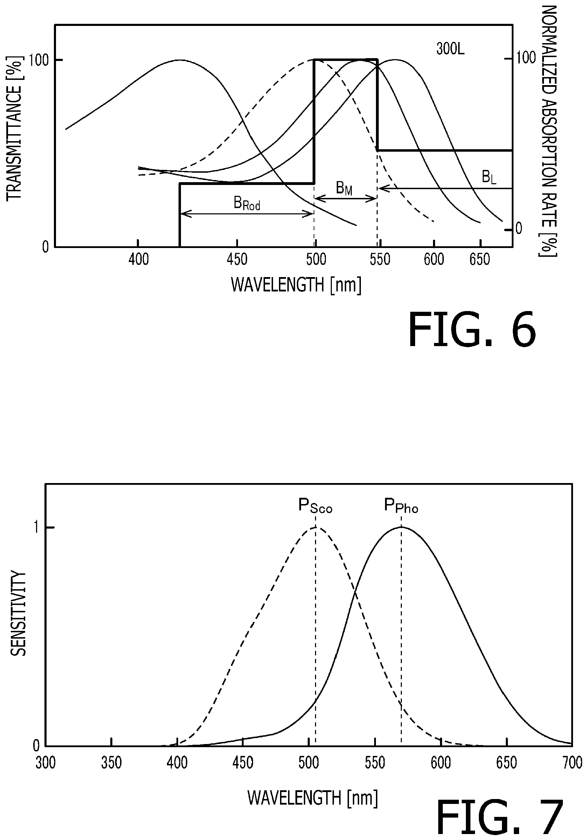

As impairments of a human visual sense, there has been known color blindness or color weakness, in which sensitivity to light in a specific wavelength band is low, and photosensitivity, in which people feel dazzled by light in a specific wavelength band. The photosensitivity is, for example, Irlen syndrome. These visual impairments are caused by higher or lower sensitivities of three cone cells (S, M, and L cone cells) or rod cells on a patient's retina compared to normal subjects. These S, M, and L cone cells are cells that respond to blue light, green light, and red light, respectively. The rod cells are cells that respond to the light intensity. The light sensitivity of a human depends on brightness of the environment, and the sensitivity in a bright environment is called photopic vision and the sensitivity in a dark environment is called scotopic vision. The photopic vision is mainly achieved by the cone cells, while the scotopic vision is mainly achieved by the rod cells (see Fig. 7). The light sensitivity in an intermediate environment between the two environments is called mesopic vision. Both the cone cells and the rod cells are responsible for the mesopic vision. As a method of correcting the human visual sense of visually impaired patients, there has been known a method using optical filters that light transmission characteristics are adjusted to the patients. The visual abnormality of the patient is suppressed when the patient wears glasses with optical filters of which the characteristics have been adjusted.

In order to make an optical filter adjusted to the patient, it is necessary to test the patient's visual characteristics (sensitivities) to various colors of light. However, since there are countless combinations of sensitivities to various lights, the test of visual characteristics is burdensome for both a testing person and the patient.

Methods for making optical filters adjusted to the patient have heretofore been known. For example, a method for making eyeglasses that classify a patient's color vision characteristics has been described in Japanese Patent Provisional Publication H06-18819 (Patent Document 1). In the method described in Patent Document 1, color vision characteristics are classified into 32 types based on test results of multiple patients' color vision characteristics. In Patent Document 1, it is examined which of the 32 types of color vision characteristics the patient's color vision characteristics correspond to. By determining the characteristics of the optical filter based on the test results, the patient's color vision abnormality is suppressed.

### SUMMARY OF INVENTION

### PROBLEMS TO BE SOLVED BY INVENTION

In the color vision testing method described in Patent Document 1, it is determined which of the predetermined types of color vision characteristics the patient's color vision characteristics correspond to. Therefore, there is a problem that accurate test results are unable to be obtained for patients with color vision characteristics that do not correspond to any of the predetermined types, or for patients having intermediate color vision characteristics between a plurality of types. In the test of visual characteristics described in Patent Document 1, the color vision characteristics are classified according to the wavelength band to which the cone cells are sensitive, but the sensitivity of the rod cells is not taken into consideration. Therefore, the test of visual characteristics described in Patent Document 1 is unable to examine abnormalities in visual characteristics caused by influences of the rod cells. In addition, the method does not take into account the photopic, scotopic, and mesopic visions, which are affected by the combined sensitivity of the cone cells and the rod cells.

The present invention was made in view of the above circumstances, and the purpose of the present invention is to provide a method for testing visual characteristics, a method for determining the characteristics of an optical filter, an optical filter, an optical element set for testing visual characteristics, and a test image for testing visual characteristics, with less load on the subject of the test.

### MEANS FOR SOLVING PROBLEMS

According to aspects of the present disclosure, there is provided a method for testing visual characteristics, comprising a first determining step of determining whether a first test condition is satisfied when a subject is looking at a test image through an optical element for testing, and at least one of a changing step of changing the optical element for testing to be placed between the subject and the test image from one element to another among a plurality of optical elements included in an optical element set prepared for the optical element for testing, and a providing step of providing the subject with a particular image as the test image. The particular image includes a first image area containing a color to which rod cells of a human are sensitive, the first image area being placed on the particular image in such a manner that light coming from the first image area forms an image on a region outside a central fovea of a retina of the subject when the subject is looking at a center of the particular image. The optical element set includes a plurality of first optical elements configured to transmit light of a first wavelength band to which the rod cells are sensitive within a visible light band, the plurality of first optical elements having respective different transmittances in the first wavelength band. The plurality of first optical elements have a common upper limit of the first wavelength band, the upper limit of the first wavelength band being set between a wavelength at which an absorption spectrum of S cone cells of the human and an absorption spectrum of the rod cells intersect each other, and a wavelength at which the absorption spectrum of the rod cells and an absorption spectrum of M cone cells of the human intersect each other. When the method for testing visual characteristics comprises the changing step, the first determining step specifies, from among the plurality of first optical elements, a first optical element that satisfies the first test condition when the subject is looking at the test image through the first optical element placed between the subject and the test image.

When the method for testing visual characteristics includes the changing step, an first optical element that satisfies the first test condition of the subject is specified from among the plurality of first optical elements configured to transmit light of a first wavelength band to which the rod cells are sensitive. With this configuration, it is possible to test sensitivities of the rod cells of the subject. Further, when the method for testing visual characteristics includes the providing step, light coming from the first image area forms an image on a region outside a central fovea of a retina of the subject. Since the first image area contains a color to which the rod cells are sensitive, it is possible to test sensitivities of the rod cells of the subject.

According to aspects of the present disclosure, there is provided a method for determining characteristics of optical filter including a determining step of determining transmittance, in the first wavelength band, of an optical filter used for adjusting light intensity based on transmittance, in the first wavelength band, of the first optical element, the first optical element being determined to be satisfied the first test condition.

According to aspects of the present disclosure, there is provided an optical filter that transmittance in the first wavelength band of the optical filter being set to transmittance, in the first wavelength band, determined by the method for testing visual characteristics.

According to aspects of the present disclosure, there is provided an optical element set for testing visual characteristics including a plurality of optical elements. The optical element set includes a plurality of first optical elements configured to transmit light of a first wavelength band to which rod cells of a human are sensitive within a visible light band, the plurality of first optical elements having respective different transmittances in the first wavelength band. The plurality of first optical elements have a common upper limit of the first wavelength band, the upper limit of the first wavelength band being set between a wavelength X_{S-Rod} at which an absorption spectrum of S cone cells of the human and an absorption spectrum of the rod cells intersect each other, and a wavelength X_{Rod-M} at which an absorption spectrum of the rod cells and an absorption spectrum of M cone cells of the human intersect each other.

According to aspects of the present disclosure, there is provided a test image for testing visual characteristics, including a first image area including a color to which rod cells of a human are sensitive, the first image area being placed in such a manner that light emitted from the first image area forms an image on a region outside a central fovea of a retina of a subject when the subject is looking at a center of the test image.

### EFFECT OF THE INVENTION

According to aspects of the present disclosure, there is provided a method for testing visual characteristics, an optical filter, an optical element set for testing visual characteristics and a test image for testing visual characteristics with less burden on the subject.

### BRIEF DESCRIPTION OF THE ACCOMPANYING DRAWINGS

[Fig. 1] Fig. 1 is a schematic diagram of a testing system of visual characteristics according to aspects of the present disclosure.
[Fig. 2] Fig. 2 is an example of absorption spectra of cone cells (S cone cells, M cone cells, L cone cells) and rod cells of a human.
[Fig. 3] Figs. 3A, 3B and 3C are band widths of a color filters according to aspects of the present disclosure.
[Fig. 4] Fig. 4 is an example of a test image according to aspects of the present disclosure.
[Fig. 5] Fig. 5 is a flow chart of a method for testing visual characteristics according to aspects of the present disclosure.
[Fig. 6] Fig. 6 is an example of the characteristics of the correction filter according to aspects of the present disclosure.
[Fig. 7] Fig. 7 indicates a photopic vision and a mesopic vision of a human.

### DESCRIPTION OF THE EMBODIMENT

Hereinafter, an illustrative embodiment according to aspects of the present disclosure will be described referring to the accompanying drawings.

### [Testing System of Visual Characteristics]

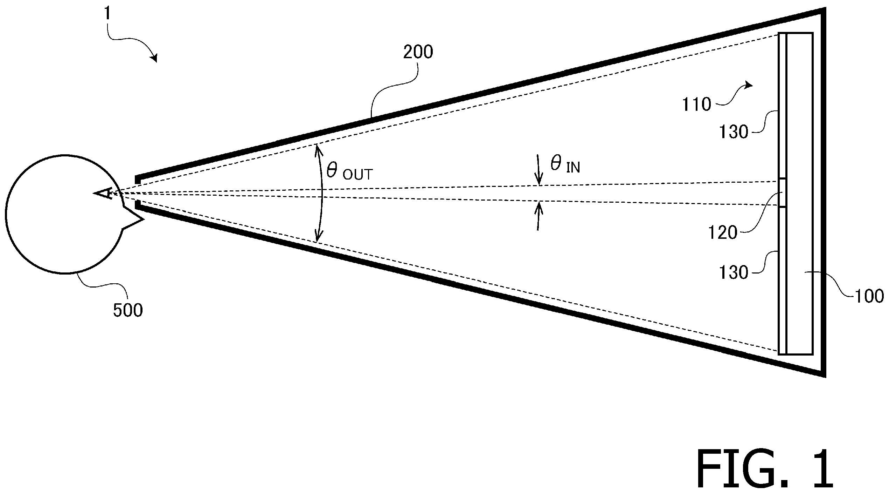

Fig. 1 is a schematic diagram of a testing system of visual characteristics 1 for performing a test of visual characteristics according to one embodiment of the invention. The testing system of visual characteristics 1 is equipped with a display 100, a light shielding hood 200, a color filter 300, and is used to test the visual characteristics of a subject 500.

The display 100 is, for example, a liquid crystal display or a CRT (Cathode Ray Tube) display. The display 100 displays a test image 110. The display 100 is used to show the test image 110 to the subject 500. The display 100 is not limited to the liquid crystal display that displays an image based on image signals. For example, the display 100 is equipped with a film on which the test image 110 is printed and a backlight that illuminates the film. The test image 110 may be presented to the subject 500 by illuminating the film with illumination light.

The display 100 is covered with the light shielding hood 200. The light shielding hood 200 is configured to prevent the test image 110 from being illuminated by external light and changing the brightness and color of the test image 110 as seen by the subject 50. The inside of the light shielding hood 200 should be black, which absorbs light, in order to prevent light from reflecting and affecting the test of visual characteristics.

The color filter 300 is worn over eyes of the subject 500 like glasses during the test of visual characteristics. The color filter 300 may be placed between the eyes of the subject 500 and the test image 110, and its form is not limited. For example, the color filter 300 may be positioned on the front surface of the display 100 to cover the test image 110, or it may be placed between the subject 500 and the display 100 and within the light shielding hood 200. A plurality of color filters 300 having different characteristics may be used. The plurality of color filters 300 are examples of a set of optical elements for the test of visual characteristics.

In the test of visual characteristics, the subject 500 views the test image 110 through the color filter 300. The subject 500 then changes the color filter 300 to test how much glare the subject 500 perceives on the test image 110 (i.e., degree of photosensitivity) and how the subject perceives the color of the test image 110 (i.e., color vision). Since the color filter 300 is used to test the subject 500, it should have the characteristic of transmitting light in the visible light band. The color filter 300 may have any transmittance in bands other than the visible light band.

When the visual characteristics of the subject 500 are tested by the test of visual characteristics, a correction filter that corrects the visual characteristics of the subject 500 can be produced based on the test results. The test results are used not only to produce the correction filter, but also to adjust the brightness and color of the lighting equipment or monitors such as TVs and mobile terminals used by the subject 500 to match the visual characteristics of the subject 50.

### [Color Filter]

Color filter 300 is a bandpass filter that transmits only light in a specific wavelength band. The bandwidth of the bandpass filter is determined based on the characteristics of human cone and rod cells.

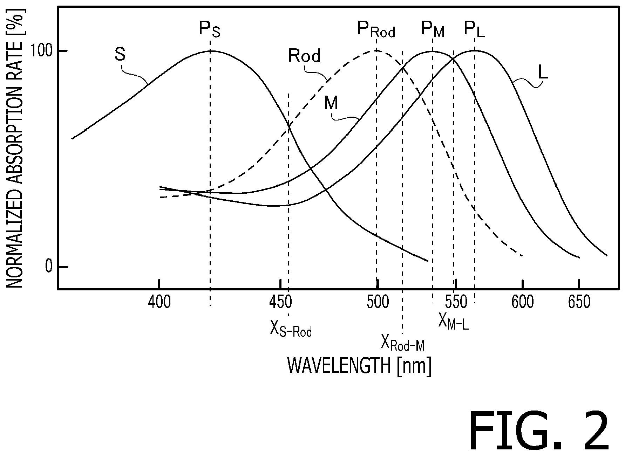

Fig. 2 shows an example of absorption spectra of human cone cells (S cone cells, M cone cells, and L cone cells) and rod cells. The horizontal axis in Fig. 2 indicates the wavelength of light, and the vertical axis indicates absorption rates of the cone cells and rod cells. In Fig. 2, "S," "M," "L," and "Rod" indicates The S cone cells, The M cone cells, The L cone cells, and The rod cells, respectively. Each absorption spectrum shown in Fig. 2 is normalized by the maximum value of absorptivity. The higher the absorptivity of each of the cone cells and the rod cells, the more sensitive it is to light. The S cone cells have a maximum sensitivity at a wavelength of about 420 nm. The M cone cells have maximum sensitivity at a wavelength of about 534 nm. The L cone cells have a maximum sensitivity at a wavelength of about 564 nm. The rod cells have a maximum sensitivity at a wavelength of about 498 nm. The wavelength at which each of cone and rod cells has the maximum sensitivity varies depend on individuals.

The S, M, and L cone cells are sensitive to light in the blue, green, and red wavelength bands, respectively. However, when a human recognizes colors, the intensity of the light absorbed by the S, M, and L cone cells does not necessarily correspond to B, G, and R components in an RGC color space. In the following, a two-color experiment by Edwin Herbert Land on human color vision will be described. In the two-color experiment, a slide (a positive film) R of an image of a subject photographed through a red filter and a slide (a positive film) G of an image of a subject photographed through a green filter are used. The images on the slides R and G are , respectively, monochrome images represented in a grayscale according to the light intensity distributions of photographed subject images.

The slide R is illuminated with red light and projected onto a screen. The slide G is illuminated with white light and projected onto the screen. As a result, a composite projected image is displayed on the screen, in which a projected image with only a red wavelength component and a projected image with a white wavelength component are superimposed on each other. The light intensity of the two projection images projected on the screen is adjusted according to a subject who watches the composite projection image. The subject thenperceives the composite projection image as a full-color image including blue and green colors. In other words, in this two-color experiment, the subject perceives blue and green colors in the projected image superimposed by the red and white lights.

In the two-color experiment, when the slide R is illuminated with a white light and the slide G is illuminated with a green light, and a projected image with a white wavelength component and a projected image with a green wavelength component are superimposed on the screen to create a composite projection image, the subject perceives blue and red colors for the composite projection image superimposed by the white and green lights.

Thus, the visual characteristics of the human is not merely linear addition of the three primary colors (red, green, and blue) of lights, but is significantly related to the cognitive functions of the brain. In the two-color experiment, the human perceives full-color for projected images superimposed by the red and white lights or by the white and green lights. Therefore, the M cone cells and L cone cells, which are sensitive to the green light and the red light, respectively, are thought to be used by the human to recognize full-color.

In addition, since the cone cells are mainly responsible for the photopic vision, it is thought that in bright areas, the human recognizes colors by the M and L cone cells, and recognizes the brightness of light by the remaining S cone cells. On the other hand, since rod cells are mainly responsible for scotopic vision, it is thought that in dark area, humans recognize the brightness of light by these rod cells. Therefore, a human with high sensitivities to the S cone cells and the rod cells, which are used to recognize the brightness of light, experiences the symptoms of photosensitivity, in which the human feels dazzled by the light. In addition, color weakness or color blindness occurs in a human whose the sensitivities of the M cone cells and the L cone cells used for color perception (i.e., sensitivity to the green light and sensitivity to the red light) are different from each other.

The characteristics of the color filter 300 are determined to be suitable for testing the degree of photosensitivity and the difference in sensitivity to green light and red light. The color filter 300 includes filter 300Rod suitable for testing the degree of photosensitivity, and filters 300M and 300L suitable for testing sensitivity difference between the sensitivity to the green light and the sensitivity to the red light.

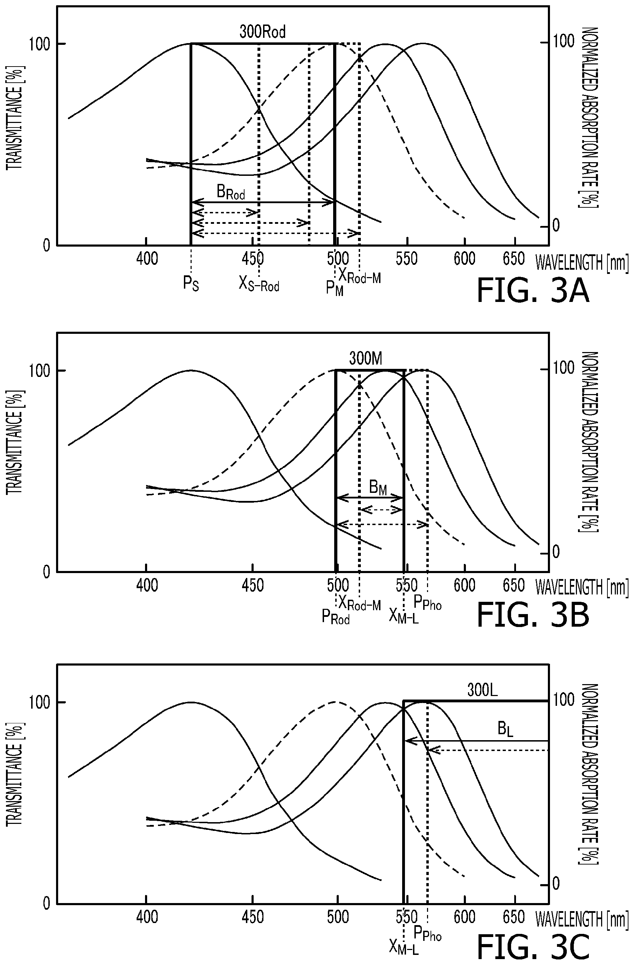

Figs. 3A-3C show bandwidths that a plurality of color filters 300 transmit lights therethrough, respectively. Figs. 3A-3C respectively show the bandwidths B_{Rod}, B_{M}, and B_{L} of the three types of filters 300 Rod, 300M, and 300L, respectively. The horizontal axes in Figs. 3A-3C indicate wavelengths of lights, and the vertical axes indicate the normalized transmittances of respective filters. The absorption spectra of the cone cells and the rod cells are overlaid in Figs. 3A-3C, and the vertical axes on the right sides of Figs. 3A-3C indicate the absorption rates, respectively.

The filter 300Rod is used to test the degree of photosensitivity of the subject 500, and has a characteristic of transmitting light in the wavelength band to which the S cone cells and the rod cells are sensitive.

The filter 300Rod has the characteristics of transmitting light in the wavelength band to which the S cone and the rod cells are sensitive, as shown by the solid line in Fig. 3A. The filter 300Rod transmits light in the wavelength band above the peak wavelength Ps (about 420 nm) of the sensitivity of the S cone cells and below the peak wavelength P_{Rod} (about 498 nm) of the sensitivity of the rod cells. In other words, the lower limit of the bandwidth B_{Rod} of filter 300Rod is Ps and the upper limit is P_{Rod}.

In order to transmit more light in the wavelength band that the S cone cells are sensitive, the filter 300Rod may transmit light at a wavelength below the peak wavelength Ps of sensitivity of the S cone cells. However, light with the wavelength below the peak wavelength Ps of the S cone cells has little effect on the color perceived by a human, although it makes the human feel the brightness of light. Therefore, if the subject 500 has symptoms of photosensitivity, the filter 300Rod that does not transmit light with a wavelength below the peak wavelength Ps of the sensitivity of the S cone cells is able to effectively suppress the symptoms of photosensitivity of the subject 500 without significantly affecting the color vision of the subject 500.

In addition, the filter 300Rod is used to test the light sensitivities of the S cone and the rod cells of the subject 500, and the upper limit of the bandwidth B_{Rod} is not limited to the peak wavelength P_{Rod} (about 498 nm) of the rod cells. Fig. 3A shows another example of the upper limit of the bandwidth B_{Rod} of the filter 300Rod and the bandwidth B_{Rod} in that case by dotted lines.

For example, the upper limit of the bandwidth B_{Rod} of the filter 300Rod may be the wavelength X_{Rod-M} (about 515 nm) at which the absorption spectrum of the rod cells and the absorption spectrum of the M cone cell intersect each other. This wavelength X_{Rod-M} is longer than the peak wavelength P_{Rod} and shorter than the peak wavelength P_{M}. In a band with a wavelength longer than X_{Rod-M}, the sensitivity of the rod cells is relatively low and the sensitivity of the M cone cells is relatively high. Therefore, if the upper limit of the bandwidth B_{Rod} of the filter 300Rod is longer than the wavelength X_{Rod-M}, the degree of light absorbed by the M cone cells will be larger, and it may not be possible to accurately test for photosensitivity due to the rod cells.

In addition, the upper limit of the bandwidth B_{Rod} of the filter 300Rod may be shorter than the peak wavelength P_{Rod} (about 498 nm) of sensitivity of the rod cells. For example, the upper limit of the bandwidth B_{Rod} of the filter 300Rod may be the wavelength XS-Rod (about 453 nm) at which the absorption spectrum of the S cells and the absorption spectrum of the rod cells intersect each other. This wavelength XS-Rod is longer than the peak wavelength Ps and shorter than the peak wavelength P_{Rod}. In the band with a wavelength shorter than the XS-Rod wavelength, the sensitivity of the rod cells is relatively low and the sensitivity of the S cone cells is relatively high. Therefore, if the upper limit of the bandwidth B_{Rod} of the filter 300Rod is shorter than the wavelength XS-Rod, the degree of light absorbed by the S cone cells will be larger, and it may not be possible to accurately test for photosensitivity due to rod cells.

In addition, in order to accurately test photosensitivity caused by rod cells, the bandwidth B_{Rod} of the filter 300Rod should include a wavelength band close to the peak sensitivity wavelength P_{Rod} of rod cells. Therefore, the upper limit of the bandwidth B_{Rod} of filter 300Rod should be shorter than the peak sensitivity wavelength P_{Rod} of the rod cell, but it should not be too far from the peak wavelength P_{Rod}. For example, when the difference between the peak sensitivity wavelength P_{Rod} of the rod cells and the wavelength X_{Rod-M} at which the absorption spectrum of the rod cells and the absorption spectrum of the M cone cells are intersect each other is Δ, by setting the upper limit of the bandwidth B_{Rod} of filter 300Rod within the range of P_{Rod} ±Δ, it is possible to accurately test for photosensitivity caused by rod cells.

The filter 300M is a filter for testing the sensitivity to green light of the subject 500 and has a characteristic of transmitting light in the wavelength band to which M cone cells are sensitive.

The filter 300M, as shown by the solid line in Fig. 3B, transmits only light at wavelengths above the peak sensitivity wavelength P_{Rod} (about 498 nm) of the rod cells and below the wavelength X_{M-L} (about 548 nm) where the absorption spectrum of the M cone cells intersects that of the L cone cells. This wavelength X_{M-L} is longer than the peak wavelength P_{M} and shorter than the peak wavelength P_{L}. In other words, the lower limit of the bandwidth B_{M} of the filter 300M is the wavelength P_{Rod} and the upper limit is the wavelength X_{M-L}.

The characteristics of the filter 300M are used to make a correction filter that corrects the visual characteristics of the subject 500. If the subject 500 wishes to emphasize the brightness of the correction filter, the upper limit of the bandwidth B_{M} of filter 300M may be set to the wavelength P_{Pho} (about 570 nm), which is the wavelength of maximum sensitivity for the photopic vision shown in Fig. 7 instead of the wavelength X_{M-L}. With this configuration, it is possible to suppress the effect on the photopic vision due to the correction filter made with using the characteristics of filter 300M.

In order to increase the degree of light in the wavelength band to which the M cone cells are sensitive among the light transmitted through the filter 300M, the lower limit of the bandwidth B_{M} of the filter 300M may be set to the wavelength X_{Rod-M} (about 515 nm), which is the wavelength at which the absorption spectrum of the rod cells and that of M cone cells intersect each other. The bandwidth B_{M} of the filter 300M in this case is shown in Fig. 3B as a dotted line. In this case, only light in the wavelength band that is relatively more sensitive to the M cone cells than to the rod cells or other cone cells passes through the filter 300M.

The rod cells are cells that respond to intensity of light and do not affect the color perception (color vision) of the subject. Therefore, even if the lower limit of the bandwidth B_{M} of the filter 300M is set to the peak sensitivity wavelength P_{Rod} of the rod cells, the sensitivity of the M cone cells to green light can be tested. In addition, when the lower limit of the bandwidth B_{M} is set to the peak sensitivity wavelength P_{Rod} of the rod cells, compared to when the lower limit is set to the wavelength X_{Rod-M}, the test image 110 that the subject 50 watches becomes brighter. This makes it easier for the subject 500 to watches the test image 110.

The filter 300L is a filter for testing the sensitivity of the subject 500 to red light. The filter 300L has the characteristic of transmitting light in the wavelength band to which the L cone cells are sensitive.

The filter 300L transmits only light at wavelengths above the wavelength X_{M-L} (about 548 nm), where the absorption spectrum of the M cone cells intersects that of L cone cells, as shown by the solid line in Fig. 3C. In other words, the lower limit of the bandwidth BL of the filter 300L is the wavelength X_{M-L}.

In the wavelength band shorter than the wavelength X_{M-L}, the sensitivity of the L cone cells becomes lower and the sensitivity of the M cone cells becomes dominant. Therefore, if the lower limit of the bandwidth B_{L} of the filter 300L is set shorter than the wavelength X_{M-L}, the sensitivity to red light by the L cone cells may not be accurately tested.

If the subject 500 wants to emphasize the brightness of the correction filter made based on the characteristics of the filter 300L, the lower limit of the bandwidth B_{L} of filter 300L may be the wavelength P_{Pho} (about 570 nm), which is the wavelength of maximum sensitivity of the photopic vision shown in Fig. 7, instead of the wavelength X_{M-L}.

It should be noted that the filters 300Rod, 300M, and 300L need not be perfectly rectangular as shown in Figs 3A-3C. The characteristics of the filter 300Rod need only be such that the light transmitted through the filter 300Rod is dominant in the wavelength band to which the S cone and rod cells are sensitive, and light in wavelength bands other than bandwidth B_{Rod} need not be completely blocked. The characteristics of the filter 300M need only be such that the light transmitted through the filter 300M is dominant in the wavelength band to which the M cone cells are sensitive, and light in wavelength bands other than bandwidth B_{M} need not be completely blocked. The characteristics of the filter 300L need only be such that the light transmitted through the filter 300L is dominant in the wavelength band to which the L cone cells are sensitive, and light in wavelength bands other than bandwidth B_{L} need not be completely blocked.

In addition, the color filter 300 includes a plurality of filters with different transmittance for each of the three filters 300Rod, 300M, and 300L, which have different bandwidths. In detail, the color filter 300 has 10 different filters with transmittance in the band varying from 10% to 100% in 10% increments for each of the filters 300Rod, 300M, and 300L, respectively. In other words, the color filter 300 contains a total of 30 different filters with different bandwidths or transmittances. The transmittance within the bandwidth of the color filter 300 need not differ in 10% increments. By decreasing the increments of transmittance and increasing the number of the color filters 300, the sensitivity of each cone and rod cell of the subject 500 can be tested more accurately, but the time and load required for the test will increase. On the other hand, by increasing the increments of transmittance and decreasing the number of color filters 300, the time and load required for the test can be reduced, but the accuracy of the test results for the sensitivity of each the cone and rod cell of the subject 500 will be smaller.

In addition, the characteristics of the filters 300Rod, 300M and 300L shown in Fig. 2 are set based on the absorption spectrum of each of the cone cell and rod cell normalized by the maximum values, but the embodiment of the invention is not limited to this configuration. For example, the characteristics of the filters 300Rod, 300M, 300L characteristics may be set based on the absorption spectrum of each of the cone cells and rod cell that is not normalized by the maximum values. If the absorption spectra of each of the cone and rod cells are not normalized by the maximum values, the maximum value of each absorption spectrum is not 100% and varies between cells. In this case, the peak wavelengths P_{S}, P_{Rod}, P_{M}, and P_{L} of each absorption spectrum are the same as the peak wavelengths P_{S}, P_{Rod}, P_{M}, and P_{L} when they are normalized at their maximum values. P_{Rod}, P_{M}, and P_{L} are the same as those of the maximum. On the other hand, the wavelengths at which the two absorption spectra intersect (e.g., the wavelength X_{S- Rod}, the wavelength X_{Rod-M}, and the wavelength X_{M-L}) are different from those when normalized by the maximum values. By using such absorption spectra that are not normalized by the maximum values, the differences in sensitivity between cells can be taken into account in the characteristic of each of the filter 300Rod, 300M, and 300L.

### [Test Image]

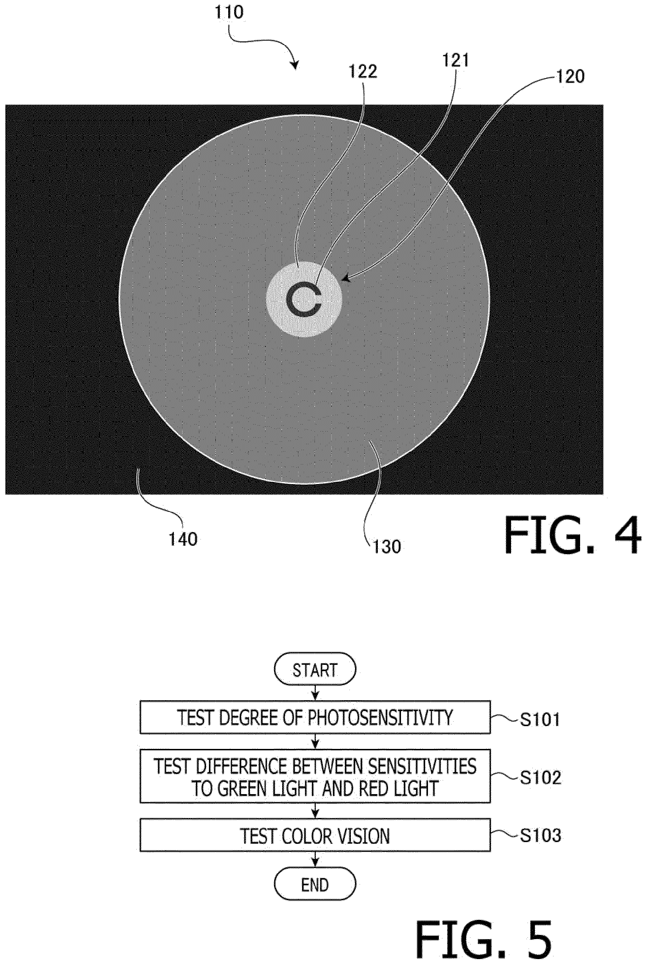

Next, the test image 110 displayed on the display 100 will be explained. Fig. 4 shows an example of the test image 110. The test image 110 is an example of a provided image of the present application.

The test image 110 includes a circular inner area 120 located near the center of the test image 110 and an outer area 130 around it. The inner area 120 and the outer area 130 of the test image 110 have colors different from each other. In the example shown in Fig. 4, the outer area 130 has a circular shape, and a peripheral area 140 further outside of the outer area 130 is black.

The inner area 120 is a region corresponding to a central fovea on a human retina. The size of the inner area 120 is set so that light coming from the inner area 120 forms an image within the central fovea. The size of the inner area 120 is set such that the apex angle θ_{IN} of a cone with the inner area 120 as a base and eyes of the subject 500 as a vertex is about 2 degrees. This apex angle θ_{IN} of about 2 degrees corresponds to the size of the field of view by the central fovea (i.e., the viewing angle). The diameter of the inner area 120 depends on the distance between the subject 500 and the display 100 in the testing system of visual characteristics 1. The size of the inner area 120 should be set so that light coming from the inner area 120 forms an image in the central fovea, and the apex angle θ_{IN} need not be exactly 2 degrees.

The outer area 130 corresponds to an area surrounding the central fovea on the human retina. The size of the outer area 130 is set in such a manner that light coming from the outer area 130 forms an image on the rod cells located outside the central fovea. The size of the outer area 130 is set in such a manner that an apex angle θ_{OUT} (see Fig. 1) of a cone with the outer area 130 as a base and the eyes of the subject 500 as a vertex is about 40 degrees (see Fig. 1). The rod cells are located mostly around ±20 degrees when the central fovea is the center of the visual field (0 degrees). Therefore, the outer area 130 is preferably set in such a manner that the apex angle θ_{OUT} is 40 degrees or more. The shape of the outer area 130 is not limited to circular. When the display screen of the display 100 is rectangular, all areas of the display screen other than the inner area 120 may be the outer area 130.

In the central fovea on the human retina, the M cone cells, which are sensitive to green light, and the L cone cells, which are sensitive to red light, are located in large numbers. The central fovea also contains few S-cone and rod cells. On the other hand, the S, M, and L cone cells and the rod cells are located in the region outside the central fovea.

A human's eyesight is higher when the central fovea is used. When a human sees objects, images, or letters, the human recognizes a shape and color of the observed object mainly by using the M and L cone cells located in the central fovea. In other words, the human can recognize colors using only the M and L cone cells. In addition, humans recognize not only color but also brightness by using the S and M cone cells and the rod cells around the central fovea. Therefore, it is possible to test a human's color vision by a visual test targeting the M cone cells and the L cone cells in the central fovea. In addition, the degree of photosensitivity can be tested by examining the S cone cells and rod cells around the central fovea.

The inner area 120 of the test image 110 is the area used for testing color vision of the M and L cone cells and contains chromatic colors. On the other hand, the outer area 130 is preferably achromatic (i.e., magnitudes of the R, G, and B components in the RGB color space are the same). This is because if the outer area 130 is colored, the color of the outer area 130 may affect the color vision test using the inner area 120. The color of the outer area 130 is not limited to achromatic colors, but have to contain colors to which the rod cells are sensitive. For example, the color of the outer area 130 is a color other than black (i.e., all of the magnitudes of the R, G, and B components are zero). The color of the outer area 130 may also be white. The outer area 130 need not be a uniform color throughout, but may include areas of relatively low or high brightness.

The inner area 120 includes at least two divided areas 121 and 122. At least one of magnitudes of the R component and the G component are different between the divided area 121 and the divided area 122. The B components of the divided areas 121 and 122 have arbitrary magnitudes. For example, the magnitude of the B components may be the same or different between divided area 121 and divided area 122. Furthermore, the magnitude of the B component in divided area 121 and divided area 122 may be zero. The inner area may include three or more divided areas of different colors. In addition, multiple types of the test images 110 with different numbers, shapes, and colors of divided areas of the inner areas 120 may be used for visual test.

In addition, if one of the divided areas 121 and 122 has an R component (i.e. the magnitude of the R component is not zero), the R component of the other of the divided areas 121 and 122 may be zero. Also, if one of the divided areas 121 and 122 has a G component (i.e. if the magnitude of the G component is non-zero), the G component of the other may be zero.

The two divided areas 121 and 121 of the inner area 120 are set in such a manner that a normal person who does not have color blindness can clearly recognize the difference between the two colors when looking at the two divided areas 121, 122.

The brightness of the color of the outer area 130 is set in such a manner that a normal person, who does not have photosensitivity, does not feel dazzled by the test image 110.

### [Method of Visual Test]

Next, a method of testing visual characteristics using the testing system of visual characteristics 1 will be described. Fig. 5 shows a flowchart of the method of testing visual characteristics in the embodiment of the present invention.

### [Processing step S101 in Fig. 5]

In processing step S101, the degree of photosensitivity of the subject 500 is tested. In other words, the degree to which the S cone and rod cells of the subject 500 are more sensitive than those of a normal person is tested.

An inspector of the visual characteristics displays the test image 110 on the display 100 and presents it to the subject 500.

The subject 500 wears one of the multiple filters 300 and watches the test image 110 displayed on the display 100 in the light shielding hood 200. At this time, the subject 500 is looking at the center of the test image 110, i.e., the inner area 120. Thus, light coming from the outer area 130 to form an image on the retina in the region outside the central fovea, where a lot of the rod cells are located.

The subject 500 switches between 10 different filters 300Rod with different transmittance in the bandwidth B_{Rod} while observing the test image 110. Then, the subject 500 selects the filter 300Rod that did not cause glare to the test image 110. If there is more than one filter 300Rod that the subject 500 did not feel dazzled by the test image 110, the subject 500 selects the filter 300Rod with the highest transmittance. The condition in which the subject 500 does not feel dazzled by the test image 110 is an example of the first test condition of this application.

The brightness of the test image 110 is set such that a person who does not have photosensitivity does not feel dazzled. Therefore, if the degree of photosensitivity of the subject 500 is small, or if the subject500 does not have photosensitivity, the subject 500 will select the filter 300Rod, which has a relatively high transmittance. On the other hand, if the degree of photosensitivity of subject 500 is high, the subject 500 will select the filter 300Rod with a relatively low transmittance.

The glare that the subject 500 feels dazzled is subjective to the subject 500. Therefore, the transmittance of the filter 300Rod is not necessarily determined only by the magnitude of sensitivity of the S cone cells and rod cells, but also includes the influence of the subject's preference for vision.

### [Processing Step S102 in Fig. 5]

In processing step S102, it is tested whether the subject 500 has low sensitivity to either green light or red light. In other words, the difference between the sensitivity of the M cone cells and the L cone cells of the subject 500 is tested.

This test is performed, for example, using the well-known Ishihara color test chart. The Ishihara color test chart is a chart commonly used in testing for color deficiency. The Ishihara color test chart is colored in such a way that letters (e.g., numbers) can be recognized when a color vision of a subject is normal or when color deficiency of the subject is properly corrected. Since the Ishihara color test chart is well known to those skilled in the art, explanation thereof is omitted herein.

### [Processing Step S103 in Fig. 5]

In processing step S103, the color vision of the subject 500 is tested. In detail, the sensitivity of one of the M and L cone cells that is determined to be sensitive in step S102 is tested compared to the sensitivity of the other.

The subject 500 wears either one of the filters 300M and 300L and observes the test image 110 displayed on the display 100 in the light shielding hood 200. When it is determined in step S102 that the M cone cells of the subject 500 have higher sensitivity than the L cone cells, the subject 500 wears the filter 300M. When it is determined in step S102 that the L cone cells of the subject 500 have higher sensitivity than the M cone cells, the subject 500 wears the filter 300L.

The subject 500, with either one of the filters 300M and 300L wore, watches the center of the test image 110, that is, the inner area 120. As a result, the light coming from the inner region 120 is imaged in the central fovea on the retina, where a lot of the M and L cone cells are located.

The subject 500 observes the test image 110 while switching 10 types of the filters 300M or the filters 300L having different transmittances of the bandwidth. Then, the subject 500 selects the filter 300M or the filter 300L that allow the subject 500 to clearly recognize the color difference between the multiple divided areas 121 and 122 of the inner area 120. When there are a plurality of the filters 300M or a plurality of the filters 300L that allow the subject 500 to clearly recognize the color difference between the plurality of divided areas 121 and 122, the filter 300M or the filter 300L that the subject 500 can most clearly recognize the color difference among them is selected. A condition in which the subject 500 can clearly recognize the difference in color between the divided areas 121 and 122 is an example of the second test condition of the present application.

For example, when the sensitivities of the M cone cells of the subject 500 are higher than the sensitivities of the L cone cells, the subject 500 wears the filter 300M in step S103. Then, the subject 500 selects the filter 300M that allow the subject 500 to most clearly recognize the difference in color between the divided areas 121 and 122. If the difference between the sensitivities of the M cone cells and the sensitivities of the L cone cells are small, the subject 500 selects the filter 300M having a relatively high transmittance of the bandwidth B_{M}. Further, as the sensitivities of the M cone cells are larger than the sensitivities of the L cone cells, the subject 500 selects the filter 300M having a low transmittance of the bandwidth B_{M}.

The difference in color between the divided areas 121 and 122 recognized by the subject 500 is due to the subjectivity of the subject 500. Therefore, the filter 300M or the filter 300L selected by the subject 500 is not necessarily determined only by the difference in sensitivity between the M cone cells and the L cone cells, and includes the influence of the subject 500's preference on the color vision.

In addition, the subject 500 may have lower (or higher) sensitivity than that of a healthy subject for both the M cone cells and the L cone cells. Therefore, in step S103, both the test with the subject 500 wearing the filter 300M and the test with the subject 500 wearing the filter 300L may be performed. Thereby, it is possible to test how much the sensitivities of the M cone cells and the L cone cells of the subject 500 differs from the sensitivities of the M cone cells and the L cone cells of a healthy person.

Further, if it is known in advance that the subject 500 does not have photosensitivity, the step S101 may be omitted. Further, when only the degree of photosensitivity of the subject 500 is tested, only the step S101 may be executed.

### [Correction filter]

Once the visual characteristics of the subject 500 are tested by the method of testing visual characteristics, the test results are used to make a correction filter that corrects the visual characteristics of the subject 500. The correction filter may be any one that changes the transmission spectrum, and there is no particular limitation on the material or the principle of changing the transmission spectrum. The correction filter is an example of the optical filter of this application.

The characteristics of the correction filter are determined based on the characteristics of the filter 300Rod selected in step S101 and at least one of the characteristics of the filter 300M and the filter 300L selected in step S103.

The transmittance of the correction filter in the bandwidth B_{Rod} which is the same as the bandwidth of the filter 300Rod is set to substantially the same transmittance as that of the selected filter 300Rod.

The transmittance of the correction filter in the bandwidth B_{M} which is the same as the bandwidth of the filter 300M is set to substantially the same transmittance as the selected filter 300M when the filter 300M is selected in step S103. On the other hand, when the filter 300M is not selected in step S103 (for example, when only the filter 300L is selected), the transmittance of the bandwidth B_{M} of the correction filter is set to approximately 100%.

The transmittance of the correction filter in the bandwidth B_{L} which is the same as the bandwidth of the filter 300L is set to substantially the same transmittance as the selected filter 300L when the filter 300L is selected in step S103.On the other hand, when the filter 300L is not selected in step S103 (for example, when only the filter 300M is selected), the transmittance of the bandwidth B_{L} of the correction filter is set to approximately 100%.

Fig. 6 shows an example of the characteristics of the correction filter. The horizontal axis of Fig. 6 indicates the wavelength of light, and the vertical axis indicates the light transmittance of the correction filter. The correction filter shown in Fig. 6 is a filter in a case that the filter 300Rod having the bandwidth B_{Rod} transmittance of 30% is selected in step S101, it is determined, in step S102, that a sensitivity of the subject 500 to red light is higher than a sensitivity to green, and the filter 300L having a transmittance of 50% in the bandwidth B_{L} is selected in step S103. In Fig. 6, the absorption spectrum of each cone cells and the rod cells are shown in an overlapping manner, and the vertical axis on the right side of Fig. 6 shows the absorption rate of each cone cells and the rod cells.

As shown in Fig. 6, the transmittance of the correction filter in the bandwidth B_{Rod} is 30%, the transmittance in the band B_{M} is 100%, and the transmittance in the bandwidth B_{L} is 50%. In the example shown in Fig. 6, the transmittance in a band of wavelength shorter than the bandwidth B_{Rod} is almost 0%. This is to reduce the glare felt by the subject 500 who has photosensitivity. However, since the photosensitivity of the subject 500 is corrected by reducing the transmittance in the bandwidth B_{Rod} of the correction filter, the transmittance in the band of wavelength shorter than the bandwidth B_{Rod} does not have to be 0%. In the example shown in Fig. 6, in the bandwidth B_{L}, the transmittance in the wavelength X_{M-L} (about 548 nm) and above are set to 50%. However, light with wavelengths longer than about 650 nm has low absorption in both the cone and rod cells and has little effect on a human color vision. Therefore, the transmittance of the correction filter for light with wavelengths longer than around 650 nm may be set to any value.

In the example shown in Fig. 6, there is no gap between the three bandwidths B_{Rod}, B_{M}, and B_{L}. However, depending on the characteristics of the filters to be used, gaps may occur between the three bandwidths B_{Rod}, B_{M}, and B_{L}. For example, when the upper limit of the bandwidth B_{Rod} of the filter 300Rod is the wavelength P_{Rod} and the lower limit of the bandwidth B_{M} of the filter 300M is the wavelength X_{Rod-M}, the gap occurs between the bandwidths B_{Rod} and the bandwidth B_{M}. In this case, the transmission between the wavelength P_{Rod} and the wavelength X_{Rod-M} of the correction filter may be set to a transmission between the transmission in bandwidth B_{Rod} and the transmission in bandwidth B_{M}, or it may be set to about 0%.

In this way, the correction filter is produced based on the test result of the visual characteristics, and the subject 500 can correct the visual characteristics by wearing the correction filter. Specifically, this correction filter can suppress photosensitivity caused by the S cone cells and rod cells of the subject 500. In addition, this correction filter can reduce color vision deficiency caused by the sensitivity difference or sensitivity ratio between the M and L cone cells of the subject 500.

Further, in the present embodiment, the subject 500 observes the test image 110 while switching the plurality of filters 300Rods having different transmittances (up to 10 types of filters 300Rods in this embodiment), thereby the degree of photosensitivity can be tested. Further, in the present embodiment, since the filter 300Rod transmits light that the S cone cells and the rod cells are sensitive, the degree of photosensitivity caused by the rod cells can be tested.

Further, the subject 500 can test the color vision of the subject 500 by observing the test image 110 while switching the plurality of filters 300M having different transmittances or the plurality of filters 300L having different transmittances (up to 10 types of filters 300M or up to 10 types of filters 300L in this embodiment). Further, in the present embodiment, since the filter 300M transmits the light that the M cone cells are sensitive and the filter 300L transmits the light that the L cone cells are sensitive, color vision abnormalities due to sensitivity difference or ratio between the M cone cells and the L cone cell can be tested.

Furthermore, in the present embodiment, as the color filter 300, 10 types of filters having different transmittances from each other are prepared for each of the filter 300Rod, the filter 300M, and the filter 300L. However, in the present embodiment, it is not necessary to test all combinations of these color filters 300 for testing the visual characteristics of the subject 500. In the present embodiment, the visual characteristics of the subject 500 are tested by a unique measurement method of visual characteristics in consideration of the characteristics of each cone cells and rod cells, and 20 types of color filters 300 are used for the test (That is, 10 types of the filters 300Rod, and 10 types of the filters 300M or 300L). Therefore, it is possible to suppress the burden on the subject 500 and the inspector by the test of visual characteristics.

In the present embodiment, as the test image 110, a plurality of divided areas 121 and 122 having different colors are provided in the inner region 120 corresponding to the central fovea, and the outer area 130 around the divided areas 120 is achromatic. As a result, with using only one test image 110, the color vision characteristics of the subject 500 (that is, the characteristics corresponding to the difference or ratio of the sensitivities of the M and L cone cells) and the degree of photosensitivity (that is, characteristics corresponding to the sensitivity of the S cone cells and rod cells). Therefore, it is possible to suppress the burden on the subject and the inspector caused by the test of visual characteristics, and to test the accurate visual characteristics in consideration of the characteristics of each cone cells and rod cells.

The above is a description of an exemplary embodiment of the invention. The embodiments of the invention are not limited to those described above, and various variations are possible within the scope of the technical concept of the invention. For example, the embodiments explicitly indicated by way of example in the specification or combinations of obvious embodiments as appropriate are also included in the embodiments of the invention.

### EXPLANATION OF SYMBOLS

- 1: a testing system of visual characteristics
- 100: a display
- 200: a light-shielding hood
- 300: a color filter
- 500: a subject

## Claims

1. A method for testing visual characteristics, comprising:
a first determining step of determining whether a first test condition is satisfied when a subject is looking at a test image through an optical element for testing; and
at least one of:
a changing step of changing the optical element for testing to be placed between the subject and the test image from one element to another among a plurality of optical elements included in an optical element set prepared for the optical element for testing; and
a providing step of providing the subject with a particular image as the test image,
wherein the particular image includes a first image area containing a color to which rod cells of a human are sensitive, the first image area being placed on the particular image in such a manner that light coming from the first image area forms an image on a region outside a central fovea of a retina of the subject when the subject is looking at a center of the particular image,
wherein the optical element set includes a plurality of first optical elements configured to transmit light of a first wavelength band to which the rod cells are sensitive within a visible light band, the plurality of first optical elements having respective different transmittances in the first wavelength band,
wherein the plurality of first optical elements have a common upper limit of the first wavelength band, the upper limit of the first wavelength band being set between a wavelength at which an absorption spectrum of S cone cells of the human and an absorption spectrum of the rod cells intersect each other, and a wavelength at which the absorption spectrum of the rod cells and an absorption spectrum of M cone cells of the human intersect each other, and
wherein, when the method for testing visual characteristics comprises the changing step, the first determining step specifies, from among the plurality of first optical elements, a first optical element that satisfies the first test condition when the subject is looking at the test image through the first optical element placed between the subject and the test image.

2. The method for testing visual characteristics according to claim 1,
wherein the first test condition is a condition that the subject does not feel dazzled by the test image when the subject is looking at the test image.

3. The method for testing visual characteristics according to claim 1 or claim 2,
wherein, when the method for testing visual characteristics includes the changing step, and the plurality of first optical elements include more than one first optical element satisfying the first test condition, the first determining step specifies a first optical element having a highest transmittance from among the more than one first optical element satisfying the first test condition.

4. The method for testing visual characteristics according to any one of claims 1 to 3,
wherein the particular image further includes a second image area, the second image area being placed on the particular image in such a manner that light coming from the second image area forms an image on a region within the central fovea of the retina of the subject when the subject is looking at a center of the second image area,
wherein the second image area contains at least two divided areas having respective different colors,
wherein, with respect to at least one component of an R component and a G component in an RGB color space, the at least two divided areas have respective different magnitudes of the at least one component, and respective B components of the at least two divided areas have arbitrary magnitudes, and
wherein the method for testing visual characteristics further comprises a second determining step of determining whether a second test condition is satisfied when the subject is looking at the second image area.

5. The method for testing visual characteristics according to claim 4,
wherein the second test condition is a condition that the subject clearly recognizes a difference in color between the at least two divided areas when the subject is looking at the second image area.

6. The method for testing visual characteristics according to claim 4 or claim 5,
wherein the optical element set further includes:
a plurality of second optical elements configured to transmit light of a second wavelength band to which M cells of the human are sensitive; and
a plurality of third optical elements configured to transmit light of a third wavelength band to which L cells of the human are sensitive,
wherein the plurality of second optical elements have respective different transmittances in the second wavelength band,
wherein the plurality of third optical elements have respective different transmittances in the third wavelength band, and
wherein the second determining step determines whether the second test condition is satisfied when the subject is looking at the second image area through one optical element selected from among the plurality of second optical elements and the plurality of third optical elements.

7. The method for testing visual characteristics according to any one of claims 1 to 6,
wherein the absorption spectrum of the S cone cells is an absorption spectrum of the S cone cells of the human normalized by a maximum value thereof,
wherein the absorption spectrum of the rod cells is an absorption spectrum of the rod cells of the human normalized by a maximum value thereof, and
wherein the absorption spectrum of the M cone cells is an absorption spectrum of the M cone cells of the human normalized by a maximum value thereof

8. A method for determining characteristics of optical filter including a determining step of determining transmittance, in the first wavelength band, of an optical filter used for adjusting light intensity based on transmittance, in the first wavelength band, of the first optical element, the first optical element being determined to be satisfied the first test condition in the first determining step of the method for testing visual characteristics according to any one of claim 1 to claim 7.

9. The method for determining characteristics of optical filter according to claim 8,
wherein transmittance, in the first wavelength band, of the optical filter is determined to be transmittance, in the first wavelength band, of the first optical element that satisfied the first test condition.

10. The method for determining characteristics of optical filter according to claim 6, further including a determining step of determining at least one of:
transmittance, in the second wavelength band, of an optical filter used for adjusting light intensity based on transmittance, in the second wavelength band, of the second optical element which is determined to be satisfied the second test condition in the second determining step of the method for testing visual characteristics including the changing step, and
transmittance, in the third wavelength band, of an optical filter used for adjusting light intensity based on transmittance, in the third wavelength band, of the third optical element which is determined to be satisfied the second test condition in the second determining step of the method for testing visual characteristics including the changing step.

11. The method for determining characteristics of optical filter according to claim 10,
wherein transmittance, in the second wavelength band, of the optical filter is determined to be transmittance, in the second wavelength band, of the second optical element that satisfied the second test condition when the second optical element that satisfied the second test condition is determined in the second determining step, and
wherein transmittance, in the third wavelength band, of the optical filter is determined to be transmittance, in the third wavelength band, of the second third element that satisfied the second test condition when the third optical element that satisfied the second test condition is determined in the second determining step.

12. An optical filter, transmittance in the first wavelength band of the optical filter being set to transmittance, in the first wavelength band, determined by the method for testing visual characteristics according to claim 8 or claim 9.

13. An optical filter,
wherein, when transmittance in the second wavelength band is determined by the method for testing visual characteristics according to claim 10 or claim 11, transmittance in the second wavelength band of the optical filter is set to the determined transmittance in the second wavelength band, and
wherein, when transmittance in the third wavelength band is determined by the method for testing visual characteristics according to claim 10 or claim 11, transmittance in the third wavelength band of the optical filter is set to the determined transmittance in the third wavelength band.

14. An optical element set for testing visual characteristics including a plurality of optical elements,
wherein the optical element set includes a plurality of first optical elements configured to transmit light of a first wavelength band to which rod cells of a human are sensitive within a visible light band, the plurality of first optical elements having respective different transmittances in the first wavelength band, and
wherein the plurality of first optical elements have a common upper limit of the first wavelength band, the upper limit of the first wavelength band being set between a wavelength X_{S-Rod} at which an absorption spectrum of S cone cells of the human and an absorption spectrum of the rod cells intersect each other, and a wavelength X_{Rod-M} at which an absorption spectrum of the rod cells and an absorption spectrum of M cone cells of the human intersect each other.

15. The optical element set for testing visual characteristics according to claim 14,
wherein, when difference between the wavelength X_{Rod-M} at which the absorption spectrum of the S cone cells and the absorption spectrum of the rod cells intersect each other and a wavelength P_{Rod} at which the rod cells have a highest sensitivity is set to Δ, the upper limit of the first wavelength band is set within a wavelength range P_{Rod}±Δ.

16. The optical element set for testing visual characteristics according to claim 14 or claim 15,
wherein the upper limit of the first wavelength band is set to the wavelength P_{Rod} at which the rod cells have a highest sensitivity.

17. The optical element set for testing visual characteristics according to claim 14 or claim 15,
wherein the upper limit of the first wavelength band is set to about 498nm.

18. The optical element set for testing visual characteristics according to any one of claim 14 to claim 17,
wherein the plurality of first optical elements have a common lower limit of the first wavelength band, the lower limit of the first wavelength band being set to a wavelength Ps at which the S cone cells have a highest sensitivity.

19. The optical element set for testing visual characteristics according to any one of claim 14 to claim 17,
wherein the plurality of first optical elements have a common lower limit of the first wavelength band, the lower limit of the first wavelength band being set to about 420nm.

20. The optical element set for testing visual characteristics according to any one of claim 14 to claim 19,
wherein the absorption spectrum of the S cone cells is an absorption spectrum of the S cone cells of the human normalized by a maximum value thereof,
wherein the absorption spectrum of the rod cells is an absorption spectrum of the rod cells of the human normalized by a maximum value thereof, and
wherein the absorption spectrum of the M cone cells is an absorption spectrum of the M cone cells of the human normalized by a maximum value thereof.

21. The optical element set for testing visual characteristics according to any one of claim 14 to claim 20,
wherein the optical element set includes a plurality of second optical elements and a plurality of third optical elements, the plurality of second optical elements being configured to transmit light of a second wavelength band to which M cells of the human are sensitive, the plurality of third optical elements being configured to transmit light of a third wavelength band to which L cells of the human are sensitive,
wherein the plurality of second optical elements have respective different transmittances in the second wavelength band, and
wherein the plurality of third optical elements have respective different transmittances in the third wavelength band.

22. The optical element set for testing visual characteristics according to claim 21,
wherein the plurality of second optical elements have a common upper limit of the second wavelength band, the upper limit of the second wavelength band being set to a wavelength X_{M-L} at which an absorption spectrum of the M cone cells and an absorption spectrum of the L cone cells intersect each other.

23. The optical element set for testing visual characteristics according to claim 22,
wherein the absorption spectrum of the M cone cells is an absorption spectrum of the M cone cells of the human normalized by a maximum value thereof, and
wherein the absorption spectrum of the L cone cells is an absorption spectrum of the L cone cells of the human normalized by a maximum value thereof.

24. The optical element set for testing visual characteristics according to claim 21,
wherein the plurality of second optical elements have a common upper limit of the second wavelength band, the upper limit of the second wavelength band being set to about 548 nm.

25. The optical element set for testing visual characteristics according to claim 21,
wherein the plurality of second optical elements have a common upper limit of the second wavelength band, the upper limit of the second wavelength band being set to a wavelength P_{Pho} at which a photopic vision of a human has a highest sensitivity.

26. The optical element set for testing visual characteristics according to any one of claim 21 to claim 25,
wherein the plurality of second optical elements have a common lower limit of the second wavelength band, the lower limit of the second wavelength band being set between a wavelength P_{Rod} at which the rod cells have a highest sensitivity and a wavelength X_{Rod-M} at which an absorption spectrum of the rod cells and an absorption spectrum of the M cone cells intersect each other.

27. The optical element set for testing visual characteristics according to claim 26,
wherein the lower limit of the second wavelength band is set to the wavelength P_{Rod} at which the rod cells have a highest sensitivity.

28. The optical element set for testing visual characteristics according to claim 26,
wherein the lower limit of the second wavelength band is set to about 498 nm.

29. The optical element set for testing visual characteristics according to claim 26,
wherein the lower limit of the second wavelength band is set to the wavelength X_{Rod-M} at which an absorption spectrum of the rod cells and an absorption spectrum of the M cone cells intersect each other.

30. The optical element set for testing visual characteristics according to claim 26,
wherein the lower limit of the second wavelength band is set to about 515 nm.

31. The optical element set for testing visual characteristics according to any one of claim 21 to claim 30,
wherein the plurality of third optical elements have a common lower limit of the third wavelength band, the lower limit of the third wavelength band being set to a wavelength X_{M-L} at which an absorption spectrum of the M cone cells and an absorption spectrum of the L cone cells intersect each other.

32. The optical element set for testing visual characteristics according to claim 31,
wherein the absorption spectrum of the M cone cells is an absorption spectrum of the M cone cells of the human normalized by a maximum value thereof, and
wherein the absorption spectrum of the L cone cells is an absorption spectrum of the L cone cells of the human normalized by a maximum value thereof.

33. The optical element set for testing visual characteristics according to claim 31,
wherein the lower limit of the third wavelength band is set to about 548 nm.

34. The optical element set for testing visual characteristics according to any one of claim 21 to claim 30,
wherein the plurality of third optical elements have a common lower limit of the third wavelength band, the lower limit of the third wavelength band being set to a wavelength P_{Pho} at which a photopic vision of a human has a highest sensitivity.

35. A test image for testing visual characteristics, including a first image area including a color to which rod cells of a human are sensitive, the first image area being placed in such a manner that light coming from the first image area forms an image on a region outside a central fovea of a retina of a subject when the subject is looking at a center of the test image.

36. The test image for testing visual characteristics according to claim 35,
wherein a color of the first image area is achromatic.

37. The test image for testing visual characteristics according to claim 35 or claim 36, including a second image area, the second image area being placed in such a manner that light coming from the second image area forms an image on a region within the central fovea of the retina of the subject when the subject is looking at a center of the second image area,
wherein the second image area contains at least two divided areas having colors different from each other, and
wherein at least one of a magnitude of an R component and a magnitude a G component in RGB color space are different among the at least two divided areas, and magnitudes of B components of the at least two divided areas are arbitrary values, respecitvely.

38. The test image for testing visual characteristics according to claim 37,
wherein a size of the second image area is set in such a manner that the second image area is positioned within a viewing angle of the subject of 2 degrees when the subject is looking at a center of the second image area.

39. The test image for testing visual characteristics according to claim 37 or claim 38,
wherein the first image area is:
placed around the second image area; and
placed, on the test image, from outer periphery of the second image area to a position corresponding to a viewing angle of 40 degrees of the subject when the subject is looking at the second image area.

40. An optical element set for testing visual characteristics, including a plurality of optical elements,
wherein the optical element set includes a plurality of first optical elements configured to transmit light of a first wavelength band within a visible light band, the plurality of first optical elements having respective different transmittances in the first wavelength band, and
wherein the plurality of first optical elements have a common upper limit of the first wavelength band, the upper limit of the first wavelength band being set between about 453 nm and about 515 nm.

41. The optical element set for testing visual characteristics according to claim 40,
wherein the plurality of first optical elements have a common lower limit of the first wavelength band, the lower limit of the first wavelength band being set to about 420 nm.

42. The optical element set for testing visual characteristics according to claim 40 or claim 41,
wherein the optical element set includes a plurality of second optical elements and a plurality of third optical elements, the plurality of second optical elements being configured to transmit light of a second wavelength band to which M cells of the human are sensitive, the plurality of third optical elements being configured to transmit light of a third wavelength band to which L cells of the human are sensitive,
wherein the plurality of second optical elements have respective different transmittance in the second wavelength band, and
wherein the plurality of third optical elements have respective different transmittance in the third wavelength band.

43. The optical element set for testing visual characteristics according to claim 42,
wherein the plurality of second optical elements have a common upper limit of the second wavelength band, the upper limit of the second wavelength band being set between about 548 nm and about 570 nm, and
wherein the plurality of second optical elements have a common lower limit of the second wavelength band, the lower limit of the second wavelength band being set between about 498 nm and about 515 nm.

44. The optical element set for testing visual characteristics according to claim 42 or claim 43,
wherein the plurality of third optical elements have a common lower limit of the third wavelength band, the lower limit of the third wavelength band being set between about 548 nm and about 570 nm.
